# EUROPEAN PATENT APPLICATION

(11) **EP 4 549 623 A1**
(43) Date of publication of application: **07.05.2025**
(21) Application number: 23831499.1
(22) Date of filing: 28.06.2023
(51) Int. Cl.: C25B 3/25, C12M 1/40, C25B 3/05, C25B 3/09, C25B 9/00, C25B 11/02, C25B 11/04, C12N 9/02, C12N 11/14

(54) **TARGET MOLECULE REDUCTION METHOD, REDUCTION DEVICE, AND ELECTRODE**

(30) Priority: 30.06.2022 JP 2022105674
(71) Applicant: Panasonic Intellectual Property Management Co., Ltd., Kadoma-shi, Osaka 571-0057 (JP)
(72) Inventor: WAYAMA, Fumiya, Kadoma-shi, Osaka 571-0057 (JP); TSUJI, Kiyotaka, Kadoma-shi, Osaka 571-0057 (JP); HATSUGAI, Noriyuki, Kadoma-shi, Osaka 571-0057 (JP)
(74) Representative: Novagraaf International SA
(86) International application number: PCT/JP2023/023939
(87) International publication number: WO 2024/005059

(57) **Abstract**

A target molecule reduction method includes: reducing a disulfide bond that is in a target molecule present in a reaction system by using thioredoxin in a reduced form, the thioredoxin being changeable between an oxidized form and the reduced form; and changing the thioredoxin from the oxidized form to the reduced form by donating, to the thioredoxin that has changed to the oxidized form due to being oxidized by the disulfide bond in the reducing, an electron to reduce the thioredoxin, the electron being from an electrode that is connected to an external power supply outside of the reaction system, wherein the thioredoxin is immobilized on a surface of the electrode directly or via a linker, and the reducing and the changing are performed with the reaction system under a basic condition.

## Description

### [Technical Field]

The present disclosure relates to a target molecule reduction method, a disulfide bond-reducing device, and an electrode.

### [Background Art]

It is known that by immobilizing enzymes on an electrode, electron transfer between the electrode and the enzymes can be utilized for various intended usages. For example, Patent Literature (PTL) 1 discloses an enzyme electrode obtained by immobilizing a carrier, an enzyme, and a mediator on a conductive member.

### [Citation List]

### [Patent Literature]

[PTL 1] Japanese Unexamined Patent Application Publication No. 2006-058289

### [Summary of Invention]

### [Technical Problem]

The enzyme electrode disclosed in PTL 1 can be used as a sensor for observing electron transfer from the enzyme to the electrode and measuring the current value.

However, a suitable reduction method is necessary for target molecule reduction involving, e.g., using an electrode donated from an electrode to cleave a disulfide bond contained in the target molecule by way of reduction (to cause cleavage).

Thus, from the viewpoint of reducing a target molecule through, e.g., the cleavage of a disulfide bond contained in the target molecule, the present disclosure provides a more suitable reduction method, and the like.

### [Solution to Problem]

A target molecule reduction method according to one aspect of the present disclosure includes: reducing a disulfide bond that is in a target molecule present in a reaction system by using thioredoxin in a reduced form, the thioredoxin being changeable between an oxidized form and the reduced form; and changing the thioredoxin from the oxidized form to the reduced form by donating, to the thioredoxin that has changed to the oxidized form due to being oxidized by the disulfide bond in the reducing, an electron to reduce the thioredoxin, the electron being from an electrode that is connected to an external power supply outside of the reaction system, wherein the thioredoxin is immobilized on a surface of the electrode directly or via a linker, and the reducing and the changing are performed with the reaction system under a basic condition.

Furthermore, a reduction device according to one aspect of the present disclosure reduces a disulfide bond that is in a target molecule present in a reaction system, and includes: a cell that separates the reaction system from an outside; and an electrode connected to an external power supply outside of the reaction system, wherein thioredoxin that is changeable between an oxidized form and a reduced form is immobilized, directly or via a linker, on a surface of the electrode.

Furthermore, an electrode according to one aspect of the present disclosure is an electrode for a reduction device that reduces a disulfide bond that is in a target molecule present in a reaction system, wherein the electrode is connected to an external power supply outside of the reaction system, and thioredoxin that is changeable between an oxidized form and a reduced form is immobilized, directly or via a linker, on a surface of the electrode.

### [Advantageous Effects of Invention]

From the viewpoint of reducing a target molecule, the present disclosure enables providing a more suitable reduction method, and the like.

### [Brief Description of Drawings]

[FIG. 1]

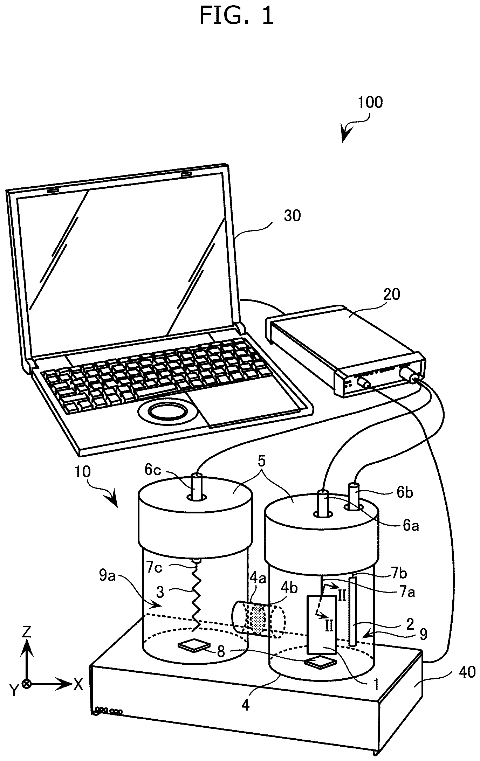

   FIG. 1 illustrates an example of the configuration of a disulfide bond-cleaving device according to the present embodiment.
[FIG. 2]

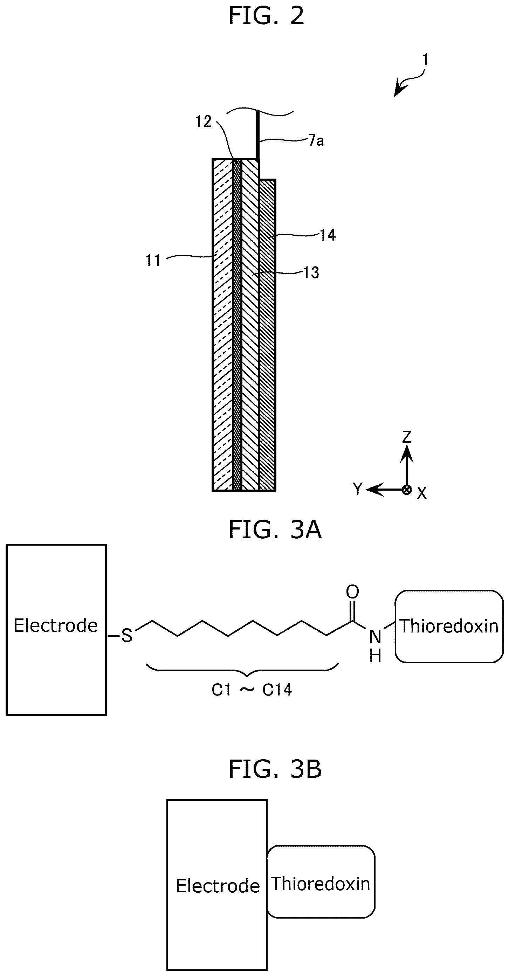

   FIG. 2 is a cross-sectional view taken along line II-II in FIG. 1.
[FIG. 3A]
   FIG. 3A is a diagram that schematically illustrates thioredoxin immobilized on an electrode.
[FIG. 3B]
   FIG. 3B is another diagram that schematically illustrates thioredoxin immobilized on an electrode.
[FIG. 4]

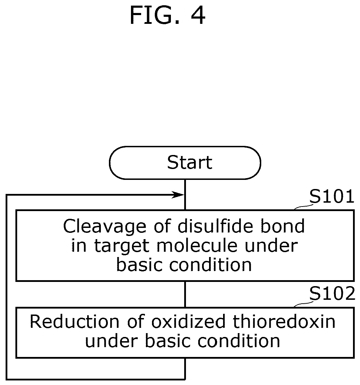

   FIG. 4 is a flow chart that illustrates an example of a method for cleaving a disulfide bond.
[FIG. 5]

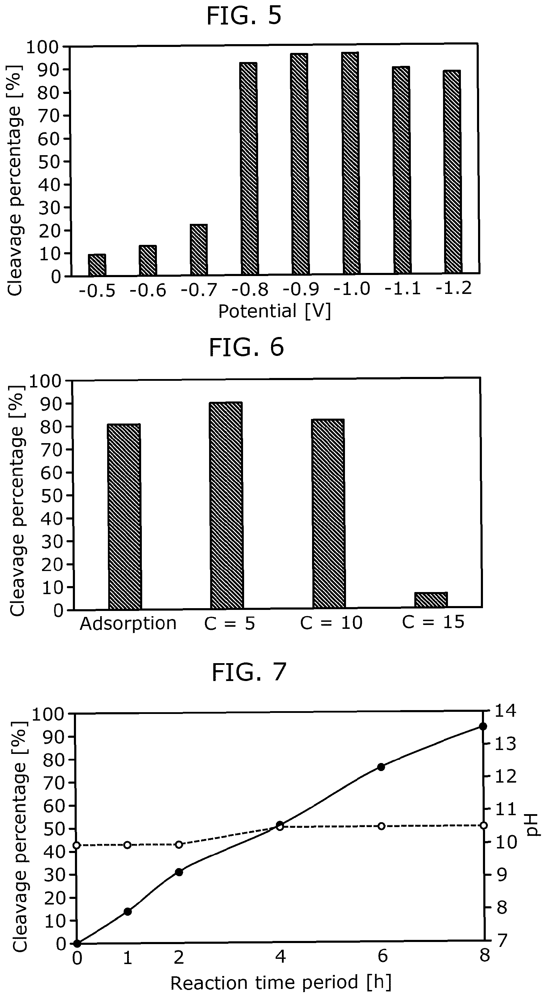

   FIG. 5 is a first drawing for describing Working Examples.
[FIG. 6]
   FIG. 6 is a second drawing for describing the Working Examples.
[FIG. 7]
   FIG. 7 is a third drawing for describing the Working Examples.
[FIG. 8]

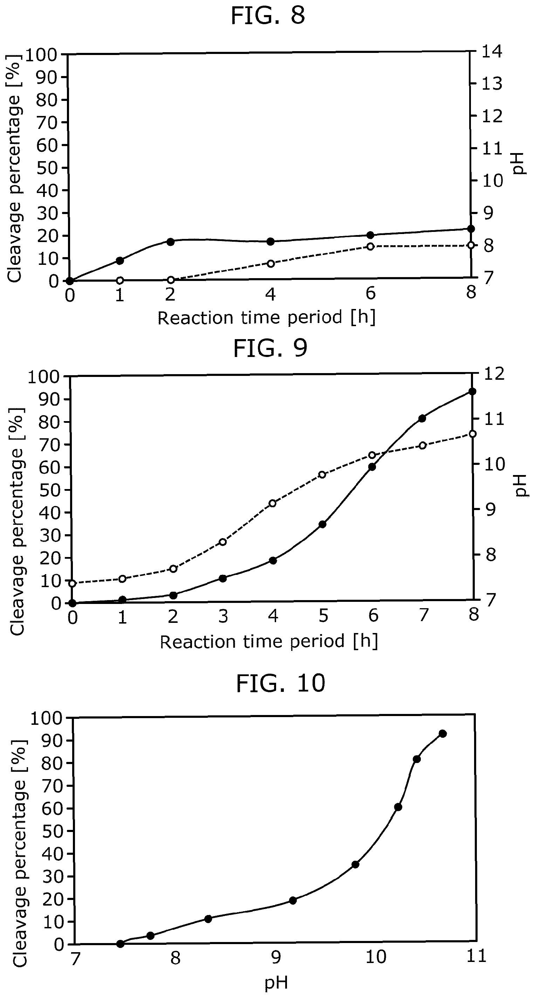

   FIG. 8 is a fourth drawing for describing the Working Examples.
[FIG. 9]
   FIG. 9 is a fifth drawing for describing the Working Examples.
[FIG. 10]
   FIG. 10 is a sixth drawing for describing the Working Examples.

### [Description of Embodiments]

### (Circumstances Leading to the Present Disclosure)

The reduction of a target molecule involving, e.g., using an electron donated from an electrode to perform cleavage by reducing a disulfide bond contained in the target molecule, necessitates a molecular species that can be used to receive the electron donated from the electrode and use this electron as reducing power to reduce the target molecule. In the above-described example, it is known that conventionally, the enzyme-protein combination of thioredoxin reductase and thioredoxin can be used as such a molecular species. Thioredoxin is changeable between the two forms of an oxidized form and a reduced form. Furthermore, reduced thioredoxin has the function of cleaving a disulfide bond contained in a target molecule. Thioredoxin that has cleaved the disulfide bond is oxidized in reciprocation for cleaving the disulfide bond, and changes to oxidized thioredoxin.

Thioredoxin reductase has the function of using an electron to reduce oxidized thioredoxin, thereby changing the oxidized thioredoxin to reduced thioredoxin. Thus, if an electron from an electrode can be donated to thioredoxin reductase, it is possible to change oxidized thioredoxin into reduced thioredoxin. In other words, it is possible to repeat the cycle of cleaving the disulfide bond contained in the target molecule, changing thioredoxin that has taken on the oxidized form into the reduced form, and again cleaving the disulfide bond contained in the target molecule. However, due to being an enzyme, thioredoxin reductase has an optimum pH, and when conditions fall outside of a certain pH range, the function of changing thioredoxin into the reduced form becomes less likely to act. More specifically, it is known that under a basic condition, the function of thioredoxin reductase deteriorates.

Furthermore, it is known to be difficult for thioredoxin reductase to directly receive an electron from an electrode, typically necessitating the presence of an electron carrier referred to as an electron mediator (or simply mediator).

On the other hand, there is an intended purpose involving utilizing the function of thioredoxin of cleaving a disulfide bond to cleave disulfide bonds in allergens (causative agents that bring about allergic reactions) contained in foods. If it is possible to cleave a disulfide bond of an allergen, it could be possible to make allergic reactions less likely due to changing or facilitating changing the three-dimensional structure of the allergen. In other words, by means of disulfide bond-cleaving treatments, technical development is underway to produce foods that contain allergens but are unlikely to cause allergic reactions. Prolamins, ovalbumin, and β-lactoglobulin, and the like are the representative allergens, but the above-described treatments can be effectively applied not only to these, but to all allergens in which disulfide bonds are present, particularly near the molecular surface.

In the process of manufacturing foods, there are cases of including a step of performing, under a basic condition, a pretreatment on food ingredients that serve as the raw food ingredients. Having the ability to also cleave a disulfide bond in this step would make it possible to simplify steps, which could also lead to decreasing food manufacturing costs.

However, as described above, in a system involving the use of thioredoxin reductase, use under a basic condition is difficult due to the function of thioredoxin reductase deteriorating. Accordingly, from the viewpoint of reducing of a target molecule by, e.g., cleaving a disulfide bond contained in the target molecule as in the above-described example, the present disclosure provides a target molecule reduction method that can be used even under a reaction condition that is a basic condition.

### (One aspect of the present disclosure)

An outline of an aspect of the present disclosure is as follows.

A target molecule reduction method according to a first aspect of the present disclosure includes: reducing a disulfide bond that is in a target molecule present in a reaction system by using thioredoxin in a reduced form, the thioredoxin being changeable between an oxidized form and the reduced form; and changing the thioredoxin from the oxidized form to the reduced form by donating, to the thioredoxin that has changed to the oxidized form due to being oxidized by the disulfide bond in the reducing, an electron to reduce the thioredoxin, the electron being from an electrode that is connected to an external power supply outside of the reaction system, wherein the thioredoxin is immobilized on a surface of the electrode directly or via a linker, and the reducing and the changing are performed with the reaction system under a basic condition.

Such a target molecule reduction method makes it possible to donate the electron from the electrode by immobilizing the thioredoxin on the electrode either directly or via a linker. The reduced thioredoxin is changed into the oxidized form each time the target molecule is reduced, and further, changes to the reduced form by means of the electron donated from the electrode. This makes it possible for the thioredoxin to repeatedly reduce the target molecule as long as the electron continues to be donated from the electrode. Furthermore, due to the thioredoxin being immobilized on the electrode, the thioredoxin is unlikely to remain in the reaction system, making the action of the thioredoxin effective in, e.g., a food processing usage. Furthermore, when the thioredoxin is immobilized on the electrode, the three-dimensional structure of the thioredoxin changes, making it possible to cleave the disulfide bond under a basic condition and to change the oxidized thioredoxin to the reduced form. Thus, from the viewpoint of cleaving the disulfide bond contained in the target molecule, it is possible to realize a method for cleaving a disulfide bond that can be used even under a reaction condition that is a basic condition.

Furthermore, for example, a target molecule reduction method according to a second aspect may be the target molecule reduction method according to the first aspect, in which the first step and the second step are performed with the reaction system under a pH condition of greater than pH 8.5.

This makes it possible to cleave the disulfide bond under a basic condition in which the pH of the reaction system is greater than pH 8.5, and to change the oxidized thioredoxin to the reduced form.

Furthermore, for example, a target molecule reduction method according to a third aspect may be the target molecule reduction method according to the first aspect or the second aspect, in which the linker includes an alkyl chain, and a total number of carbon atoms in the alkyl chain is at least 1 and at most 14.

This makes it possible to donate an electron from the electrode by immobilizing the thioredoxin on the electrode via a linker that includes an alkyl chain, the total number of carbon atoms in the alkyl chain being at least 1 and at most 14.

Furthermore, for example, a target molecule reduction method according to a fourth aspect may be the target molecule reduction method according to any one of the first to third aspects, in which the electrode includes gold, platinum, glassy carbon, or indium tin oxide (ITO).

This makes it possible to repeatedly cleave the disulfide bond by means of the electrode on which the thioredoxin is immobilized, the electrode including gold, platinum, glassy carbon, or ITO.

Furthermore, for example, a target molecule reduction method according to a fifth aspect may be the target molecule reduction method according to any one of the first to fourth aspects, in which the target molecule is at least one of a prolamin, ovalbumin, or β-lactoglobulin.

This makes it possible to use the electrode on which the thioredoxin is immobilized to repeatedly cleave a disulfide bond contained in at least one of a prolamin, ovalbumin, or β-lactoglobulin.

Furthermore, for example, a target molecule reduction method according to a sixth aspect may be the target molecule reduction method according to any one of the first to fifth aspects, in which no electron carrier that performs electron transport to and from the thioredoxin is immobilized on the electrode, and in the changing, the electron is donated from the electrode to the thioredoxin that is in the oxidized form without mediation by an electron carrier.

This makes it possible, at a time of donating an electron to the thioredoxin, to donate the electrode from the electrode to the thioredoxin without the mediation of an electron carrier. At this time, if the thioredoxin is immobilized near the electrode either directly or via a comparatively short linker whose total number of carbon atoms is, e.g., at least 1 and at most 14, electron donation efficiency is less susceptible to deterioration, even without mediation by electron carrier. Moreover, since the electron donation efficiency is limited only by the reaction speed at which the thioredoxin itself accepts the electron and is not dependent upon the reaction speed at which an electron carrier accepts the electron and the reaction speed at which the electron is released from an electron carrier, this also has the merit of facilitating improvement of the electron donation efficiency. Furthermore, due to there being fewer molecular species immobilized on the electrode, the electrode is easier to design, and product quality of the electrode can be further stabilized.

Furthermore, a reduction device according to a seventh aspect of the present disclosure reduces a disulfide bond that is in a target molecule present in a reaction system, and includes: a cell that separates the reaction system from an outside; and an electrode connected to an external power supply outside of the reaction system, wherein thioredoxin that is changeable between an oxidized form and a reduced form is immobilized, directly or via a linker, on a surface of the electrode.

Such a reduction device makes it possible to realize a reduction device that performs the target molecule reduction method according to any one of the first to sixth aspects. Thus, the reduction device achieves effects similar to those of the target molecule reduction method according to any one of the first to sixth aspects.

Furthermore, an electrode according to an eighth aspect of the present disclosure is an electrode for a reduction device that reduces a disulfide bond that is in a target molecule present in a reaction system, wherein the electrode is connected to an external power supply outside of the reaction system, and thioredoxin that is changeable between an oxidized form and a reduced form is immobilized, directly or via a linker, on a surface of the electrode.

Such an electrode makes it possible to utilize an electrode in a reduction device that performs the target molecule reduction method according to any one of the first to sixth aspects. Thus, the electrode achieves effects similar to those of the target molecule reduction method according to any one of the first to sixth aspects.

It should be noted that these general or specific aspects may be implemented as a system, a method, an apparatus, an integrated circuit, a computer program, or a computer-readable recording medium such as a CD-ROM, or may be implemented as any combination of a system, a method, an apparatus, an integrated circuit, a computer program, and a recording medium.

Hereinafter, embodiments will be described in detail with reference to the drawings.

It should be noted that each of the embodiments described shows a general or specific example of the present disclosure. The numerical values, shapes, materials, elements, the arrangement and connection of the elements, steps, the processing order of the steps, etc., indicated in the following embodiments are mere examples, and thus are not intended to limit the claims. Furthermore, among the elements described in the following embodiments, elements not recited in any one of the independent claims that indicate the broadest concepts are described as optional elements. Furthermore, the figures are schematic illustrations and are not necessarily accurate depictions. Elements which are substantially the same have the same reference signs in the figures, and duplicate description may be omitted or simplified.

The mutually orthogonal X-axis, Y-axis, and Z-axis directions illustrated in the figures will be used as appropriate in the description. In particular, the positive side in the Z-axis direction may be described as the upper side, and the negative side in the Z-axis direction may be described as the lower side.

In the present disclosure, terms indicating relationships between elements such as "parallel" and "perpendicular", terms indicating shapes of elements such as "rectangular", and numerical ranges refer not only to their strict meanings, but encompass a range of essentially equivalents, such as a range of deviations of a few percent.

In the figures of the present disclosure, dashed lines indicate the boundaries of what is not visible from the surface, as well as regions.

### [Embodiments]

Hereinafter, embodiments will be described in detail with reference to FIG. 1 to FIG. 4.

### [Disulfide bond-cleaving device]

### [1. Outline]

First, an outline of a disulfide bond-cleaving device (hereinafter, may be referred to as simply cleaving device) will be described with reference to FIG. 1, as an example of a target molecule reduction device according to an embodiment. FIG. 1 is a diagram illustrating an example of a configuration of a disulfide bond-cleaving device according to the present embodiment.

Cleaving device 100 performs electron transport between an electrode and thioredoxin by applying voltage to an electrode. For example, cleaving device 100 donates an electron from the electrode to thioredoxin immobilized on the surface of the electrode. In this way, electron exchange occurs between the electrode and a target molecule in a sample, whereby the disulfide bond in the target molecule is reduced and cleaved. For example, cleaving device 100 applies a voltage to the electrode while a sample (for example, sample solution) containing a target molecule is in a non-flowing state, whereby electron transfer takes place between the thioredoxin immobilized on the electrode, and the target molecule in the sample to reduce the target molecule. The reduced target molecule is then diffused into the liquid by switching the liquid from the non-flowing state to a flowing state. By repeatedly switching the flow state of the liquid as described above, the target molecule can be efficiently reduced throughout the entire liquid. At this time, the reaction system, i.e., the interior of the liquid containing the sample, is under a basic condition. Note that the basic condition as referred to means a condition under which the pH range is higher than neutral. Typically, neutral refers to a pH range having a certain range with pH 7 at the center (for example, pH 6 to pH 8). Thus, the basic condition in the present embodiment means a pH range that is higher than pH 8.

As used herein, a non-flowing state of a liquid means, for example, that the liquid is not agitated or shaken (for example, not subjected to an external force such as a shearing force or a vibration) and that no fluctuation or other motion is observed on the liquid surface.

### [2. Configuration]

Next, the configuration of cleaving device 100 in this embodiment will be described with reference to FIG. 1 to FIG. 3B.

As illustrated in FIG. 1, cleaving device 100 includes agitator 40 that agitates sample 9 containing a target molecule to bring the sample into a flowing state, an electrode (cathode electrode 1) on which thioredoxin that reduces the target molecule by electron transfer with the target molecule is immobilized, power supply 20 that applies a voltage to the electrode, and controller 30 that controls power supply 20 and agitator 40.

Voltage applier 10 is, for example, a three-electrode cell that includes cathode electrode 1 (also referred to as a working electrode), reference electrode 2, counter electrode 3, cell 4, lid 5, terminals 6a, 6b, and 6c, and leads 7a, 7b, and 7c. Voltage applier 10 may be a two-electrode cell that includes, for example, a working electrode (cathode electrode 1) and counter electrode 3.

Cathode electrode 1 and counter electrode 3 are made of a conductive material. The conductive material may be, for example, a carbon material, a conductive polymer material, a semiconductor, or a metal.

First, cathode electrode 1 will be described with reference to FIG. 2, FIG. 3A, and FIG. 3B. FIG. 2 is a cross-sectional view taken at line II-II in FIG. 1. FIG. 3A and FIG. 3B are diagrams schematically illustrating thioredoxin immobilized on an electrode.

Cathode electrode 1 is the electrode on which the thioredoxin is immobilized. Cathode electrode 1 includes, for example, glass substrate 11, titanium deposition layer 12 deposited on glass substrate 11, cathode substrate 13 formed on titanium deposition layer 12, and reaction layer 14 including the thioredoxin immobilized on cathode substrate 13.

As cathode substrate 13, for example, a conductive substrate such as gold, platinum, glassy carbon, or ITO (indium tin oxide) may be used. The thickness of cathode substrate 13 is not particularly limited.

No electron carrier is immobilized on cathode electrode 13. The electron carrier as referred to herein is a substance that performs electron transfer between the electrode and the thioredoxin immobilized on the electrode. Examples thereof include viologens, quinones, indophenol, and the like.

The thioredoxin immobilized on the surface (so-called cathode substrate 13) of the electrode is a protein that reduces the target molecule. As illustrated in FIG. 3A, the thioredoxin is immobilized on cathode substrate 13 by a linker that is in the form of a chain. The linker includes, for example, an alkyl chain having at least 1 and at most 14 carbon atoms. Alternatively, as illustrated in FIG. 3B, the thioredoxin may be immobilized directly on the surface of the electrode without the mediation of a linker. Note that without the mediation of a linker means that no other molecular species intervenes between the surface of the electrode and the thioredoxin. However, a case in which a part of the main chain or a side chain constituting the thioredoxin, the part being involved in the immobilization, behaves similarly to a linker is included in the concept of "without the mediation of a linker".

Reference electrode 2 is an electrode that does not react with the components in sample 9 and maintains a constant potential, and is used to control the potential difference between cathode electrode 1 and reference electrode 2 to a constant level by power supply 20. Reference electrode 2 is, for example, a silver/silver chloride electrode. Counter electrode 3 is, for example, a platinum electrode. In the present embodiment, cell 4 separates the reaction system from an outside. In cell 4, a first space in which cathode electrode 1 and reference electrode 2 are disposed, and a second space in which counter electrode 3 is disposed are connected via connector 4a. Connector 4a is provided with separator 4b, whereby some of the components among sample 9 in the first space and sample 9a in the second space do not move between the spaces.

Power supply 20 applies a voltage between cathode electrode 1 and counter electrode 3 of voltage applier 10 to control the potential between cathode electrode 1 and reference electrode 2 to a predetermined value in accordance with a control signal output from controller 30.

Controller 30 processes information for controlling the application of a voltage by power supply 20 and the movement of a motor (not illustrated) of agitator 40. Controller 30 is realized, for example, by a processor, a microcomputer, or dedicated circuitry.

Agitator 40 controls the rotation speed and the rotation time of agitating element 8 that is set in voltage applier 10 by controlling the operation of the motor in accordance with a control signal output from controller 30.

In a method for producing an immobilized thioredoxin electrode, a linker for immobilizing thioredoxin is immobilized on an electrode. The linker is dissolved in a solution in which the ratio of ethanol: acetonitrile is 1:1, and the immobilization is performed by, for example, the electrode being immersed in the solution for at least 1 hour and being allowed to stand. In order to enable the electron to be transferred from the electrode to the thioredoxin, the linker may be an alkyl chain having at most 14 carbon atoms. For example, the linker is an alkyl chain having at least 1 and at most 14 carbon atoms. In particular, the linker has a carboxyl group or an amino group at one end, and has a thiol group at the other end.

Next, the linker immobilized on the electrode and the thioredoxin are bonded. At this time, for example, the amino group in the thioredoxin and the carboxyl group in the linker are bonded by an amine coupling reaction. The thioredoxin is thus immobilized on the electrode via the linker.

Note that in the case of immobilizing the thioredoxin directly on the electrode, the step of immobilizing the linker is omitted.

### [3. Target molecule reduction method (method for cleaving disulfide bond)]

Next, as an example of the target molecule reduction method, a method for cleaving a disulfide bond is described with reference to FIG. 4. FIG. 4 is a flow chart that illustrates an example of the method for cleaving a disulfide bond.

The method for cleaving a disulfide bond contained in a target molecule cleaves a disulfide bond by reducing a target molecule contained in sample 9 by using three-electrode voltage applier 10. Three-electrode voltage applier 10 includes an immobilized thioredoxin electrode as cathode electrode 1, an anode electrode as counter electrode 3, and reference electrode 2 (S101). Step S101 is an example of the first step. Step S101 is performed under a basic condition. The surface area of the anode electrode is sufficiently greater than that of cathode electrode 1. As a consequence of cleaving the disulfide bond, the thioredoxin is oxidized and thus changes to the oxidized form. Sample 9 is an aqueous solution containing the target molecule.

Next, the thioredoxin that has changed to the oxidized form is again reduced and thus changed to the reduced form (S102). Step S102 is an example of the second step. Step S102 is performed under a basic condition. Furthermore, by repeatedly performing step S101 and step S102, cleavage of the disulfide bond contained in the target molecule can be continuously repeated. At this time, the voltage applied to sample 9 may be controlled such that the potential of cathode electrode 1 with respect to reference electrode 2 is the oxidizing potential of the thioredoxin.

### [Working Example 1: Verification of total number of carbon atoms in alkyl chain contained in linker]

### [1-1. Immobilized thioredoxin electrode 1]

Hereinafter, the method for cleaving a disulfide bond in a target molecule using the immobilized thioredoxin electrode of the present disclosure will be specifically described by way of Working Examples, but the present disclosure is not in any way limited only to the following Working Examples.

In the following Working Examples, β-lactoglobulin was used as the target molecule.

Preparation was performed by dissolving the target molecule in phosphate-buffered saline (PBS) at pH 7.4, to a concentration of 3.3 mg/mL.

For production of the immobilized thioredoxin electrode, a solution obtained by dissolving thioredoxin in phosphate-buffered saline (PBS) at pH 7.4 was added on a gold substrate and left to stand and then adsorption of the thioredoxin on the substrate was permitted, whereby the immobilized thioredoxin electrode was produced.

### [1-2. Immobilized thioredoxin electrode 2]

While immobilized thioredoxin electrode 1 described above involved producing an electrode having no linker, i.e., producing an electrode having the thioredoxin adsorbed directly thereon, immobilized thioredoxin 2 of the present example is different in the respect that thioredoxin is immobilized on the substrate via a linker having 5 carbon atoms (carboxyl alkanethiol). Thus, in the present example, first a solution of a linker having 5 carbon atoms was prepared, and a self-assembled monolayer (SAM) of the linker was formed on a substrate. Then, the amino group of the thioredoxin and the carboxyl group of the linker were bonded by an amine coupling reaction.

### [1-3. Immobilized thioredoxin electrode 3]

While immobilized thioredoxin electrode 2 described had 5 carbon atoms in the alkyl group contained in the linker, immobilized thioredoxin 3 of the present example is different in the respect that thioredoxin is immobilized on the substrate via a linker having 10 carbon atoms (carboxyl alkanethiol). Thus, in the present example, first a solution of a linker having 10 carbon atoms was prepared, and a self-assembled monolayer (SAM) of the linker was formed on a substrate. Then, the amino group in the thioredoxin and the carboxyl group in the linker were bonded by an amine coupling reaction.

### [1-4. Immobilized thioredoxin electrode 4 (Comparative Example)]

While immobilized thioredoxin electrode 2 described above had 5 carbon atoms in the alkyl group contained in the linker, immobilized thioredoxin 4 of the present example is different in the respect that thioredoxin is immobilized on the substrate via a linker having 15 carbon atoms (carboxyl alkanethiol). Thus, in the present example, first a solution of a linker having 15 carbon atoms was prepared, and a self-assembled monolayer (SAM) of the linker was formed on a substrate. Then, the amino group in the thioredoxin and the carboxyl group in the linker were bonded by an amine coupling reaction.

### [1-5. Verification results]

The target molecule solution was incorporated into cell 4, illustrated in FIG. 1, as samples 9 and 9a to set each electrode. As the electrodes, a three-electrode system of electrodes was used in which the immobilized thioredoxin electrode produced was adopted as the working electrode, a Pt (platinum) electrode was adopted as the counter electrode, and an Ag/AgCL (silver/silver chloride) electrode was adopted as the reference electrode. Next, agitating element 8 was inserted into the target molecule solution and a certain voltage, described later, was applied to the target molecule solution while rotating agitating element 8 at a certain rotation speed (rpm). Note that the reaction system used phosphate-buffered saline (PBS) at pH 7.4 as an initial condition, but it was confirmed that the pH rose to around 11 due to the hydroxy group species generated by the time of the reaction conclusion, and under that condition, it was confirmed that cleavage of the disulfide bond had progressed. In other words, the reaction system being under a basic condition means that in at least a part of the duration from the reaction starting time to the reaction conclusion time, the condition is basic (a pH range that is higher than pH 8.0). In particular, in the method for cleaving a disulfide bond according to the present disclosure, in order to apply a certain potential, it is assumed that the pH gradually rises in the case of a typical aqueous solution system; thus, enabling cleavage of the disulfide bond under a basic condition could be a merit.

To confirm cleavage of the disulfide bond in the target molecule, the sample after application of the voltage was mixed with a digestive enzyme and a reaction was permitted at 37°C for 30 min, and then analysis by SDS-PAGE was performed. The sample before applying the voltage was subjected to a similar analysis to calculate, using image analysis, a relative disulfide bond cleavage amount in the case of the sample before applying the voltage being 100%.

FIG. 5 is a graph showing the relationship between the applied potential in Working Example 1, and the relative disulfide bond cleavage amount. In FIG. 5, the horizontal axis shows the potential applied between the working electrode and the counter electrode, and the vertical axis shows the relative disulfide bond cleavage amount cleaved at each applied potential. As illustrated in FIG. 5, it was indicated that disulfide bond cleavage occurs drastically from an applied potential of -0.8 V, and the relative disulfide bond cleavage amount peaks at -1.0 V and decreases at potentials lower than -1.0 V. In the following Working Examples, the disulfide bond cleavage is performed at an applied potential of -1.0 V.

The verification results for Working Example 1 are shown in FIG. 6. FIG. 6 is a graph that illustrates the verification results for Working Example 1. In FIG. 6, the horizontal axis shows each immobilized thioredoxin electrode type, and the vertical axis shows the relative disulfide bond cleavage amount cleaved by each immobilized thioredoxin electrode type. As illustrated in FIG. 6, in immobilized thioredoxin electrode 1, on which the thioredoxin was directly adsorbed (adsorption), about 80% of the disulfide bonds were cleaved. Furthermore, in immobilized thioredoxin electrode 2, in which the thioredoxin was immobilized via a linker having 5 carbon atoms (C=5), about 90% of the disulfide bonds were cleaved. Moreover, in immobilized thioredoxin electrode 3, in which the thioredoxin was immobilized via a linker having 10 carbon atoms (C=10), about 80% of the disulfide bonds were cleaved. In contrast, it was indicated that in immobilized thioredoxin electrode 4, in which the thioredoxin was immobilized via a linker having 15 carbon atoms (C=15), the cleaved disulfide bonds failed to reach even 10%.

This result indicates that immobilized thioredoxin electrode 2 had the highest cleavage amount. It is surmised that in the electrode on which the thioredoxin was immobilized by direct adsorption, due to a lack of freedom in, e.g., the orientation of the thioredoxin, the disulfide bond cleavage efficiency decreased. On the other hand, it is surmised that in the electrode on which the thioredoxin was immobilized via the linker having 10 carbon atoms, the electron transfer efficiency from the electrode surface to the thioredoxin became lower, whereby the disulfide bond cleavage efficiency decreased.

### [Working Example 2: Verification of suitable pH range in reaction system]

Next, immobilized thioredoxin electrode 2 was used to verify the suitable pH condition for the reaction system. This is described by comparing a case of performing disulfide bond cleavage in a system capable of maintaining about pH 10 with a case of performing disulfide bond cleavage in a system capable of maintaining about pH 8 or lower. FIG. 7 is a graph illustrating the change over time of the relative disulfide bond cleavage amount when the disulfide bond cleavage is performed in a system capable of maintaining pH 10. Moreover, FIG. 8 is a graph illustrating the change over time of the relative disulfide bond cleavage amount when the disulfide bond cleavage is performed in a system capable of maintaining pH 8 or lower. Note that in FIG. 7 and FIG. 8, the solid dot symbols and solid lines indicate the relative disulfide bond cleavage amount, and the outlined dot symbols and dashed lines indicate the pH of the reaction system.

As illustrated in FIG. 7 and FIG. 8, it was indicated that in the system capable of maintaining about pH 8 or lower, except for the first 2 hours, no disulfide bond cleavage reaction proceeded, and in the system capable of maintaining about pH 10, the disulfide bond cleavage reaction proceeded throughout the entire duration of the reaction.

Furthermore, FIG. 9 is a graph illustrating the change over time of the relative disulfide bond cleavage amount in a case of performing disulfide bond cleavage under a condition involving the pH changing over time from about 7.5 to about 10.5. In FIG. 9, the solid dot symbols and solid lines indicate the relative disulfide bond cleavage amount, and the outlined dot symbols and dashed lines indicate the pH of the reaction system. Furthermore, FIG. 10 is a graph organized to illustrate the relationship between the pH of the reaction system and the relative disulfide bond cleavage amount when the condition for the reaction time period was fixed.

As illustrated in FIG. 9 and FIG. 10, it was indicated that from the time point of the pH exceeding 8.5, the disulfide bond cleavage proceeded rapidly. These results indicate that in the immobilized thioredoxin electrodes produced in the Working Examples, disulfide bond cleavage is preferably performed under a pH condition of the pH being higher than 8.5.

Although the method for cleaving a disulfide bond, the disulfide bond-cleaving device, and the electrode according to the present disclosure have been described above based on embodiments, the present disclosure is not limited to these embodiments. Various modifications to the foregoing embodiments that can be conceived by those skilled in the art as well as other forms resulting from arbitrary combinations of elements in different embodiments that do not materially depart from the essence of the present disclosure are included within the scope of the present disclosure.

### [Industrial Applicability]

The present disclosure is useful in the reduction of a target molecule.

### [Reference Signs List]

1 cathode electrode
2 reference electrode
3 counter electrode
4 cell
4a connector
4b separator
5 lid
6a terminal
6b terminal
6c terminal
7a lead
7b lead
7c lead
8 agitating element
9,9a sample
10 voltage applier
11 glass substrate
12 titanium deposition layer
13 cathode substrate
14 reaction layer
20 power supply
30 controller
40 agitator
100 cleavage device

## Claims

1. A target molecule reduction method comprising:
reducing a disulfide bond that is in a target molecule present in a reaction system by using thioredoxin in a reduced form, the thioredoxin being changeable between an oxidized form and the reduced form; and
changing the thioredoxin from the oxidized form to the reduced form by donating, to the thioredoxin that has changed to the oxidized form due to being oxidized by the disulfide bond in the reducing, an electron to reduce the thioredoxin, the electron being from an electrode that is connected to an external power supply outside of the reaction system, wherein
the thioredoxin is immobilized on a surface of the electrode directly or via a linker, and
the reducing and the changing are performed with the reaction system under a basic condition.

2. The target molecule reduction method according to claim 1, wherein
the reducing and the changing are performed with the reaction system under a pH condition of greater than pH 8.5.

3. The target molecule reduction method according to claim 1, wherein
the linker includes an alkyl chain, and
a total number of carbon atoms in the alkyl chain is at least 1 and at most 14.

4. The target molecule reduction method according to claim 1, wherein
the electrode includes gold, platinum, glassy carbon, or indium tin oxide (ITO).

5. The target molecule reduction method according to claim 1, wherein
the target molecule is at least one of a prolamin, ovalbumin, or β-lactoglobulin.

6. The target molecule reduction method according to any one of claims 1 to 5, wherein
no electron carrier that performs electron transport to and from the thioredoxin is immobilized on the electrode, and
in the changing, the electron is donated from the electrode to the thioredoxin that is in the oxidized form without mediation by an electron carrier.

7. A reduction device that reduces a disulfide bond that is in a target molecule present in a reaction system, the reduction device comprising:
a cell that separates the reaction system from an outside; and
an electrode connected to an external power supply outside of the reaction system, wherein
thioredoxin that is changeable between an oxidized form and a reduced form is immobilized, directly or via a linker, on a surface of the electrode.

8. An electrode for a reduction device that reduces a disulfide bond that is in a target molecule present in a reaction system, wherein
the electrode is connected to an external power supply outside of the reaction system, and
thioredoxin that is changeable between an oxidized form and a reduced form is immobilized, directly or via a linker, on a surface of the electrode.
